# EUROPEAN PATENT SPECIFICATION

(11) **EP 4 103 775 B1**
(45) Date of publication and mention of the grant of the patent: **21.01.2026**
(21) Application number: 21710570.9
(22) Date of filing: 12.02.2021
(51) Int. Cl.: D04H 1/728, A61Q 19/00, D01F 8/02, D06M 15/15, A61K 8/73, D01F 2/00, A61K 8/02, D01F 8/18, D01F 9/00, D01F 8/04, D01F 4/00, D01D 5/00, B32B 27/12, D01F 1/10, B32B 5/02, B32B 5/08, B32B 5/26, D01D 5/34, D01D 5/24

(54) **COSMETIC PRODUCT, PROCEDURE FOR ITS REALIZATION AND ELECTROSPINNING DEVICE**
KOSMETISCHES PRODUKT, VERFAHREN FÜR SEINE REALISIERUNG UND ELEKTROGEWINDEVORRICHTUNG
PRODUIT COSMÉTIQUE, PROCÉDURE POUR SA RÉALISATION ET DISPOSITIF D'ÉLECTROFILAGE

(30) Priority: 13.02.2020 IT 202000002842
(43) Date of publication of application: 21.12.2022
(73) Proprietor: Bakel S.P.A., 33100 Udine (IT)
(72) Inventor: GREGORIS, Raffaella, 33100 Udine (IT); MANFREDINI, Stefano, 44049 Vigarano Mainarda (IT); VERTUANI, Silvia, 44123 Ferrara (IT); ROSO, Martina, 37142 Verona (IT)
(74) Representative: Petraz, Gilberto Luigi
(86) International application number: PCT/IB2021/051193
(87) International publication number: WO 2021/161253

(56) References cited:
- WO-A1-2018/185223
- WO-A2-2010/090868
- CN-A- 109 998 932
- GB-A- 2 484 319
- US-A1- 2015 272 855
- US-A1- 2017 251 789
- US-A1- 2018 127 577

## Description

### FIELD OF THE INVENTION

The present invention concerns a cosmetic product and a method to produce it. More particularly, the invention concerns a cosmetic patch product that can be absorbed by the skin and based on polymer compounds, preferably biocompatible, made up of fibers, in particular nanofibers, preferably obtained by electrospinning, and packaged.

### BACKGROUND OF THE INVENTION

Cosmetic products to be applied directly on the skin are widely known, which are formulated in various forms compatible with the skin and/or which are absorbed by the skin, such as for example, emulsions, creams, lotions, serums, gels, powders, oils, sun milks and suchlike, or other formulations.

These products, however, can sometimes be difficult to apply on the skin, sometimes requiring prolonged application times or a prolonged massage, in order to be completely absorbed into the dermis, and sometimes leaving cosmetic residues on the fingers or hands or on any possible applicators used.

There are also cosmetic products that are applied by means of a physical support, generally fibrous, which facilitates their application on the skin, such as in particular make-up remover wipes, which are imbued with a special cleansing composition.

One disadvantage of this type of cosmetic product is that, after use, the fibrous physical support, which does not have its own cosmetic function, as well as the packaging, have to be disposed of.

Furthermore, known fibrous physical supports have a specific surface that does not allow suitable transport and release of the active ingredients.

The question also arises that, generally, cosmetic compositions for the skin, for example those to be spread, such as cream, or in general cosmetic pastes, provide to use functional compounds for the skin and compounds that are exclusively functional for the structure of the formulation.

Functional compounds for the skin are partly or completely absorbed by the skin and bring benefits to the treated parts of the body. In general, this type of compound represents the so-called active ingredients.

The compounds that are exclusively functional for the structure of the formulation contribute to obtaining the cosmetic composition, whether it be an emulsion, a serum, or fluid, an oleolyte, a water, or a gel.

The effects of a compound that is exclusively functional for the structure of the formulation may affect the storage method, or the consistency of the cosmetic composition before its use and/or at the time of use, therefore when it is spread on the skin to be treated.

For example, a compound that is exclusively functional for the structure of the formulation can make the cosmetic composition soft, fluid or viscous; it allows to obtain a gel and prevent the separation of the oily compounds from the aqueous ones in an emulsion. Furthermore, compounds that are exclusively functional for the structure of the formulation allow to preserve the cosmetic composition, especially in the presence of water.

However, the problem arises that numerous compounds used, since they are functional for the structure of the formulation, do not bring any benefit to the skin, and indeed can even lead to a further deterioration of the treated skin. In fact, some of the compounds that are exclusively functional for the structure of the formulation remain indifferent to the skin, or they can cause, for example, further drying, pore closure, or development of allergies, dermatitis, or suchlike.

Among the compounds that are exclusively functional for the structure of the formulation, we can identify, for example, silicones, petrolatums (for example paraffin), alcohols, perfumes, stabilizers, preservatives, emulsifiers, the main function of which is to give the formulation of the cosmetic composition a "silk effect" consistency in contact with the skin and/or to give stability to the oily parts, or to increase the viscosity of gel formulations and/or increase the emollient and humectant effect of the emulsions.

For example, one problem that arises with these compounds exclusively functional for the structure of the formulation is the lack of skin compatibility, preventing the skin from breathing, also leading, for example, to its progressive drying, pore closure and/or greater sensitization.

It is also known that the presence of alcohols and preservatives on the one hand allow to preserve the cosmetic composition, and on the other hand increase the sensitization of the skin in developing dermatitis, allergies, or similar pathologies.

US2015/272855 describes a cosmetic product in the form of a sheet, consisting of nanofibers obtained by electrospinning a solution comprising a water-soluble polymer, in particular polyvinyl alcohol PVA and/or polyvinylpyrrolidone PVP, and a functional material in a solvent of water and/or alcohol. The sheet product is intended to be applied to the skin for a prolonged time, typically for hours.

US2017/251789 shows a sheet-shaped cosmetic product consisting of nanofibers of water-soluble polymer material obtained by electrospinning a corresponding solution to be electrospun. The electrospun polymer material is mainly polyvinyl alcohol PVA and/or polyvinylpyrrolidone PVP. The solution also comprises a functional compound, in particular a cross-linking agent, which can be an acid, an alcohol or a derivative of formaldehyde. This sheet product is also intended to be left to act on the skin for a prolonged time.

US2018/127577 discloses the possibility of obtaining tubular-shaped fibers, but starting from emulsions, that is, by resorting to two solutions that cannot be mixed together.

WO2018/185223 shows an electrospinning device equipped with an electrospinning head consisting of two needles located coaxially. The internal needle is totally included in the external needle, the end of which is located beyond the delivery end of the internal needle. The electrospinning head has a circular delivery aperture, not configured to deliver a tubular fiber.

WO2010/090868 discloses a device comprising at least a first needle and a second needle for delivering at least a first and a second solution, respectively, during an electro spinning or electrostatic spray deposition operation. The first needle is disposed within the second needle such that the first and second solutions can be delivered and mixed.

GB2484319 describes electrospun fibers based on honey and a biocompatible polymer, which can for example comprise a polysaccharide or a synthetic polymer such as polyethylene oxide PEO, polyethylene terephthalate PET or polypropylene PP. The fibers described have an antimicrobial effect and can be used to treat skin wounds. No mention is made about a tubular shape of the fibers, or a method of device for obtaining tubular shaped fibers.

CN109998932 discloses a novel facial mask piece and preparation and using methods thereof. The mask comprises a hydrophilic nano-fiber membrane prepared through an electrospinning technology and which makes contact with the skin, on another layer made of hydrophobic base cloth. This document is silent about a tubular electrospun fiber, a method for making the same or a corresponding electrospinning device.

There is therefore a need to perfect a cosmetic product, as well as a method to produce it, that can overcome at least one of the disadvantages of the state of the art.

In particular, one purpose of the present invention is to provide a cosmetic product that can be applied simply and quickly to the skin.

Another purpose of the present invention is to provide a cosmetic product that does not have a physical support to be discarded after use.

Another purpose is to provide a cosmetic product that has a specific surface such as to optimize the transport and release of active ingredients, if there are any.

Another purpose is to produce a cosmetic product with a quantity of active ingredient that can be increased with respect to known cosmetic products.

The Applicant has devised, tested and embodied the present invention to overcome the shortcomings of the state of the art and to obtain these and other purposes and advantages.

### SUMMARY OF THE INVENTION

The present invention is set forth and characterized in the independent claims. The dependent claims describe other characteristics of the present invention or variants to the main inventive idea.

In accordance with the above purposes, in the following we describe a cosmetic patch product, a method to produce the cosmetic patch product and an eletrospinning device, which overcome the limits of the state of the art and eliminate the defects present therein.

In accordance with some embodiments, there is provided, in particular, a cosmetic patch product, preferably to be applied on the skin, made up of a membrane substrate suitable to be absorbed by the skin. The membrane substrate is electrospun. The electrospun substrate is made up of at least one fiber, preferably nanofiber, in particular in the form of non-woven fabric, obtained starting from a material able to be electrospun.

Favorably, the fiber is hollow, that is, it has a tubular shape. More specifically, the fiber has an annular shaped cross-section, and defines a containing space inside it, in which a biocompatible substance is preferably housed. The biocompatible substance can be suitable to be electrospun. For example, the biocompatible substance can be different from, or identical to, the material that makes up the substrate.

The substrate is based on a biocompatible polymer material, suitable to be electrospun, selected from a group consisting of hyaluronic acid HA, starch, amylose, cellulose, chitosan, glycogen, pectin, agar, collagen, hydroxypropyl methylcellulose HPMC and their derivatives. The substrate is also based on a second compound, used as an elctrospinning promoter during the production of the fiber, selected from pullulan and a blend of alginate with poly(oxyethylene).

Preferably, the membrane substrate comprises linear and/or cross-linked hyaluronic acid. Advantageously, the membrane substrate is made both with linear hyaluronic acid and also with cross-linked hyaluronic acid.

Preferably, the hyaluronic acid has a molecular weight of less than 10,000 Da. This molecular weight is advantageous for the complete absorbability of the hyaluronic acid in the skin.

One advantage of the cosmetic patch product according to the embodiments described here is that it is completely absorbed by the skin, together with any possible active ingredient present, for example a cosmetic active ingredient.

Another advantage is that the form in which the cosmetic patch product is presented does not imply formulations that require the presence of compounds exclusively functional for the structure, since the possible active ingredients are carried and conveyed by the membrane substrate, and the latter is completely biocompatible and able to be absorbed by the skin, without causing any risk or damage to the skin itself.

Another advantage lies in the possibility of reaching much higher topical concentrations, for example of hyaluronic acid, than those obtainable with traditional formulations. With known cosmetic products, it is practically impossible to reach high concentrations, even with hyaluronic acid, polysaccharides, collagen, hydroxypropyl methylcellulose HPMC and their low molecular weight derivatives, since the viscosity of the product increases too much and it is not possible to exceed 5-10% by weight. With this method, it is possible to apply concentrations up to 50% by weight of hyaluronic acid on the skin.

According to one aspect, a method is provided to produce a cosmetic patch product which comprises the steps of providing at least a first composition to be electrospun, providing an electrospinning device comprising an electrospinning head and a collector, applying an electric field between the electrospinning head and the collector, and feeding the composition to be electrospun through the electrospinning head, so that the electric field applied induces the formation of the electrospun fiber in order to form a membrane substrate suitable to be absorbed by the skin.

The electrospinning head comprises a delivery member which comprises at least one needle open at its free end, and the aperture of which is annular in shape and configured to generate a tubular shaped electrospun fiber.

The composition to be electrospun comprises a first compound to be elctrospun and a spinning promoter, the first compound to be electrospun is selected from a group consisting of hyaluronic acid HA, starch, amylose, cellulose, chitosan, glycogen, pectin, agar, collagen, hydroxypropyl methylcellulose and their derivatives, and the spinning promoter is selected from pullulan and a blend of alginate with poly(oxyethylene).

Preferably, the delivery member comprises a first needle and a second needle disposed coaxially to the first needle and with a diameter smaller than the diameter of the first needle. In other words, the second needle is disposed inside the first needle. In this way, the first needle has an end aperture with an annular shape, due to the presence of the second needle inside it. The latter, on the other hand, has a circular aperture.

According to some embodiments, the method provides to provide a second composition to be electrospun, and to feed the above said second composition to be electrospun, preferably at the same time as the first composition. Even more preferably, the first composition is fed toward the first needle of the delivery member, and the second composition is fed toward the second needle of the delivery member.

The electric field applied between the electrospinning head and the support has a potential difference of at least 8 kV. More preferably, the potential difference is comprised between 15 and 40 kV.

Favorably, the electrospinning head and the support are set in relative motion with respect to each other, that is, at least one of the two is set in motion.

Advantageously, at least the electrospinning step itself takes place in a controlled and constant atmosphere, more advantageously it takes place in conditions of controlled and constant temperature and relative humidity.

According to some embodiments, the composition also comprises an active ingredient. This active ingredient can, for example, be selected from polypeptides.

According to some embodiments, it is possible to provide that the active ingredient is not present in the composition to be electrospun, but that it is added to the product obtained by the electrospinning.

According to some embodiments, the composition also comprises a stabilizer, suitable to improve the stability of the fibers obtained following the electrospinning. Preferably, the stabilizer is a cross-linkable polymer.

Advantageously, the second composition to be electrospun is analogous to the first composition to be electrospun described above.

According to yet another aspect, an electrospinning device is provided which comprises an electrospinning head and a collector, a voltage generator connected both to the electrospinning head and to the collector, and at least one element for feeding a composition to be electrospun.

The electrospinning head comprises a delivery member, which in turn comprises at least one needle open at its free end, and the aperture of which is annular in shape and is configured to generate a tubular shaped electrospun fiber. The delivery member comprises a first needle and a second needle disposed coaxially to the first needle and with a diameter smaller than the diameter of the first needle. In other words, the second needle is disposed inside the first needle. In this way, the first needle has an end aperture with an annular shape, due to the presence of the second needle inside it. The second needle, on the other hand, has a circular end aperture. The second needle has an end that is disposed, with respect to the end of the first needle, in a same plane or in a plane downstream when considering the sense of feed of the compositions to be electrospun.

The electrospinning device comprises two feed elements, a first feed element connected to the first needle of the delivery member, and a second feed element distinct from the first and connected to the second needle of the delivery member. By "feed element connected to a needle of the delivery member" we mean, in the context of the present description, that they are connected in a fluidic manner, that is, in such a way as to allow the outflow of a fluid from the feed element to the needle of the delivery member.

### BRIEF DESCRIPTION OF THE DRAWINGS

These and other aspects, characteristics and advantages of the present invention will become apparent from the following description of some embodiments, given as a non-restrictive example with reference to the attached drawings wherein:
- fig. 1 is a schematic representation of an electrospinning device according to one embodiment; and
- fig. 2 is a schematic section view of a detail of the device of fig. 1, taken according to section plane II-II of fig. 1.

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

We will now refer in detail to the possible embodiments of the invention, of which one or more examples are shown in the attached drawings. Each example is supplied by way of illustration of the invention and shall not be understood as a limitation thereof. For example, one or more of the characteristics shown or described insomuch as they are part of one embodiment can be variant or adopted on, or in association with, other embodiments to produce another embodiment. It is understood that the present invention shall include all such modifications and variants.

Before describing these embodiments, we must also clarify that the present description is not limited in its application to details of the construction and disposition of the components as described in the following description using the attached drawings. The present description can provide other embodiments and can be obtained or executed in various other ways. We must also clarify that the phraseology and terminology used here is for the purposes of description only, and cannot be considered as limitative.

Unless otherwise defined, all the technical and scientific terms used here and hereafter have the same meaning as commonly understood by a person with ordinary experience in the field of the art to which the present invention belongs. Even if methods and materials similar or equivalent to those described here can be used in practice and in the trials of the present invention, the methods and materials are described hereafter as an example. In the event of conflict, the present application shall prevail, including its definitions. The materials, methods and examples have a purely illustrative purpose and shall not be understood restrictively.

All measurements are carried out, unless otherwise indicated, at 25°C (room temperature) and at atmospheric pressure. All temperatures, unless otherwise indicated, are expressed in degrees Celsius.

All percentages and ratios indicated here are understood to refer to the weight of the total composition (w/w), unless otherwise indicated.

All percentage intervals reported here are supplied with the provision that the sum with respect to the overall composition is 100%, unless otherwise indicated.

All the intervals reported here shall be understood to include the extremes, including those that report an interval "between" two values, unless otherwise indicated.

The present description also includes the intervals that derive from overlapping or uniting two or more intervals described, unless otherwise indicated.

The present description also includes the intervals that can derive from the combination of two or more values taken at different points, unless otherwise indicated.

Where water is mentioned, we mean distilled water, unless otherwise specified.

Some embodiments described here concern a cosmetic patch product, preferably to be applied on the skin, comprising an electrospun membrane substrate which is made up of at least one electrospun fiber, preferably a nanofiber, in the form of a non-woven fabric. This form is obtained by progressively depositing the fiber, during the electrospinning, on a support base. The fiber is progressively deposited on several layers which gradually overlap. It is possible to provide several fibers, which can for example be delivered simultaneously on the same support.

The nanofiber has a tubular shape, that is, a nanotube form. It is in fact hollow inside and open at its ends. In other words, the fiber comprises an internal containing space which extends longitudinally with respect to it, along its entire length.

It follows that the cross-section of the nanofiber, taken in any of its segments whatsoever, has an annular or substantially annular shape.

Preferably, the nanotube nanofiber comprises, in the internal containing space, a substance which can be, for example, an active ingredient in solution, solidified, in the form of a cream or gel, or more preferably a second nanofiber, for example also electrospun. The second nanofiber is preferably solid, that is, it has no internal containing space.

The electrospinning occurs by feeding a first composition to be electrospun under an electric field, through an electrospinning head. The nanotube fiber at exit from the electrospinning head is then deposited on a collector, on which a layer of support base material is eventually positioned. The non-woven fabric form can be obtained by means of a relative movement between the electrospinning head and the collector.

Preferably, the fiber is continuous, with a homogeneous composition and with a substantially smooth surface. The fiber is also preferably free of defects, that is, free from accumulations or drops of substrate that can form during the electrospinning. Advantageously, the electrospun fiber, with a nanotube form, has an external diameter of the order of the nanometer and micrometer, but in any case smaller than 100 µm, more preferably smaller than 50 µm, even more preferably smaller than 25 µm, at most of the order of 10 µm. The fibers can have diameters starting from tens of nanometers, for example 70-80 nm. It should be noted that both the internal and also the external diameters remain substantially unchanged along the entire fiber. By substantially unchanged diameter we mean that the diameter can undergo variations which however do not exceed 20% of the average value measured.

Fibers with these diameters have a greater surface/volume ratio than known fibers, which typically have a diameter greater than 100 µm, as well as greater mechanical and structural properties that offer greater efficiency and performance than industry standards. The large specific surface of the fibers obtained makes them particularly suitable to transport and release active ingredients.

It should be noted that the nanotube form of the fiber allows to include greater quantities of active ingredients than known fibers, but also compared to nanometric fibers that do not have a nanotube form.

Favorably, both the nanotube fiber and also the second nanofiber, if present, are made of a biocompatible polymer material suitable to be electrospun selected from a group consisting of polysaccharides, collagen, hydroxypropyl methylcellulose HPMC and their derivatives. Two different materials can be provided for the formation, respectively, of the tubular fiber and of the second fiber, provided that they are both of the biocompatible polymer type.

Preferably, the polysaccharide is selected from hyaluronic acid HA, starch, amylose, cellulose, chitosan, glycogen, pectin and agar.

Even more preferably, the substrate comprises linear and/or cross-linked hyaluronic acid. Advantageously, the substrate is made both with linear hyaluronic acid and with cross-linked hyaluronic acid. Such a mixture allows to modulate the rigidity of the yarn obtained, as well as the three-dimensional structure of the non-woven fabric film.

The hyaluronic acid can have a high mass, for example of the order of a million Dalton or even more, or alternatively have a low mass, typically of the order of 10,000 Dalton or less. The latter form is preferred, since hyaluronic acid with a molecular mass lower than or equal to 10,000 Da, in the electrospun form, once placed in contact with the skin is completely absorbable and is able to penetrate the stratum corneum, reaching the innermost layers where, thanks to its properties such as hygroscopicity, viscoelasticity and structural function, it is able to modulate the hydration of the tissues, the osmotic balance and the physical properties of the ECM.

Advantageously, the action of absorption and penetration into the skin of the membrane substrate also allows to convey possible cosmetic active ingredients associated with the membrane support toward the skin.

In particular, according to some embodiments, the cosmetic product also comprises at least one active ingredient, for example a cosmetic active ingredient, associated with the membrane substrate, preferably selected from polypeptides. In these embodiments, it becomes particularly advantageous to provide that the membrane substrate is made up of a mixture of linear hyaluronic acid with cross-linked hyaluronic acid. The presence of the latter affects the three-dimensional structure of the non-woven fabric, in particular by forming cavities, also called cages, suitable to house the molecules of the active ingredient.

The active ingredient can be added to the already electrospun membrane substrate, therefore in its non-woven fabric form, or it can be included in the composition which is then electrospun. In other words, it is possible to provide that the active ingredient is electrospun together with the material that makes up the substrate.

If the second nanofiber is also present, it is possible to provide that this be based on the same biocompatible polymer as the nanotube fiber, or be based on a different biocompatible polymer. In the event that the two nanofibers are based on the same material, it is possible to provide that only one comprises an active ingredient, or that they comprise two different active ingredients, or even that they comprise the same active ingredient but at two different concentrations, depending on requirements.

Obviously, the second nanofiber has an external diameter which substantially corresponds to the internal diameter of the nanotube fiber, that is, to the diameter of its internal space.

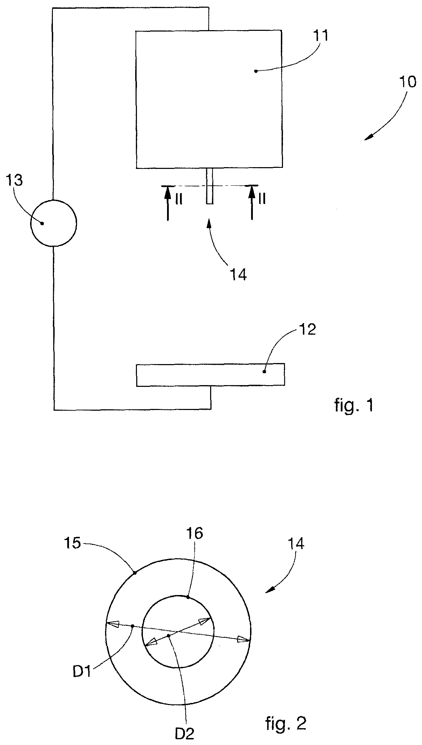

According to one aspect, fig. 1 shows an electrospinning device 10 suitable to produce the cosmetic patch product described above. This electrospinning device 10 comprises an electrospinning head 11, through which one or more compositions to be electrospun can be electrospun, a collector 12, on which the newly electrospun fiber is collected, and a voltage generator 13 connected to the electrospinning head 11 and to the collector 12 so as to produce an electric field between them. The electric field serves to induce the formation of the electrospun fiber in order to form the membrane substrate able to be absorbed by the skin.

The generator 13 can be set according to the specific properties of the composition to be electrospun, but in any case it is configured to generate an electric field of at least 8 kV between the electrospinning head 11 and the collector 12. Preferably, the electric field applied is comprised between 15 and 40 kV. The positive potential, that is, the greater one, is applied to the electrospinning head 11, while the negative potential, that is, the lower one, is applied to the collector 12.

The electrospinning head 11 comprises a delivery member 14, which in turn comprises at least a first needle 15 open at its end. The aperture of the needle 15 is annular in shape, and is configured to form a tubular shaped fiber during the electrospinning (fig. 2). Preferably, the delivery member 14 comprises two coaxial needles 15, 16, one being located inside the other.

More precisely, the delivery member 14 preferably comprises a first needle 15 and a second needle 16 with a diameter D2 smaller than the diameter D1 of the first needle 15, and located inside it so as to define the annular shape of the aperture of the first needle 15 (fig. 2). The second needle 16 advantageously has a circular aperture.

Advantageously, the open end of the second needle 16 is located in the same plane as the open end of the first needle 15, or is located downstream of the open end of the first needle 15 with respect to the sense of feed of the solutions to be electrospun. This ensures that the aperture of the first needle 15 is annular. In other words, it is advantageous for the ends of the coaxial needles 15, 16 to be in the same plane, or that the end of the second needle 16 is located outside the first needle 15, in a longitudinal sense.

According to some embodiments, the aperture of the first needle 15 has an internal diameter D1 comprised between 0.5 and 3 mm, more preferably between 0.8 and 2.5 mm, and the aperture of the second needle 16 has an internal diameter D2 comprised between 0.1 and 1 mm, more preferably between 0.2 and 0.8 mm.

The device 10 also comprises a first element for feeding a composition to be electrospun, connected to the first needle 15 so as to feed the composition as above toward it. Preferably, the device 10 also comprises a second feed element connected to the second needle 16 so as to feed a second composition toward it, preferably to be electrospun. The feed elements can comprise, for example, a tank and a pumping member connected thereto. The pumping member can be, for example, a volumetric pump.

The device 10 is suitably controlled by a control system, not shown, the settings of which are made accessible to the user, for example by means of a display.

In accordance with one aspect of the invention, there is provided a method to produce the cosmetic patch product described above. The method provides at least one step of electrospinning a first composition to be electrospun through an electrospinning head 11 provided with a delivery member 14, which in turn comprises a needle 15 open at its end and the aperture of which is annular in shape and configured to generate a tubular shaped electrospun fiber. Preferably, the delivery member 14 comprises two needles 15, 16, open at their ends, and located coaxial to each other, the two needles 15, 16 having two different diameters D1, D2 so that one can be located inside the other. Favorably, it is provided that the electrospinning step is carried out on a second composition, at the same time as on the first composition mentioned above. Advantageously, the second composition is also to be electrospun.

More precisely, the method comprises the steps of providing a first composition to be electrospun and a second composition, preferably to be electrospun; providing an electrospinning device 10 comprising an electrospinning head 11 and a collector 12, wherein the electrospinning head 11 is provided with a delivery head 14 which comprises a first needle 15 and a second needle 16 with a diameter D2 smaller than the diameter D1 of the first needle 15 and located coaxially thereto, inside it; applying an electric field between the electrospinning head 11 and the collector 12; and feeding the first composition toward the first needle 15, and the second composition toward the second needle 16, so that the electric field applied between the electrospinning head 11 and the collector 12 induces the formation of at least one first electrospun fiber, in order to form the membrane substrate able to be absorbed by the skin. Preferably, the electric field also induces the formation of a second electrospun fiber, if the second composition is to be electrospun.

It should be noted that due to the presence of the second needle 16 inside the first needle 15, the latter has an annular shaped aperture configured to generate a tubular shaped electrospun fiber (fig. 2). Therefore, the first electrospun fiber is tubular in shape.

Preferably, the electric field applied between the electrospinning head 11 and the collector 12 has a potential difference equal to at least 8 kV. More preferably, the potential difference is comprised between 15 and 40 kV.

Preferably, the first composition and the second composition are fed with volumetric flow rates comprised between 0.1 and 60 ml/hr, so as to guarantee the appropriate ratio between the materials that make up the external tubular fiber and those that form the internal fiber. Each composition can be fed independently of the other, therefore also at different times or with different flow rates.

Favorably, the electrospinning head 11 and the collector 12 are set in relative motion with respect to each other, that is, at least one of the two is set in motion.

Advantageously, at least the electrospinning step itself occurs in a controlled and constant atmosphere, more advantageously, it occurs in conditions of controlled and constant temperature and relative humidity.

According to some embodiments, the first composition to be electrospun, and preferably also the second composition, comprise a first compound to be electrospun and a spinning promoter. The spinning promoter has the function of facilitating the spinning of the first compound, in particular of establishing the electrospinning method so as to obtain regular fibers.

The first compound to be electrospun is a biocompatible polymer suitable to be electrospun and selected from a group consisting of a first polysaccharide, collagen, hydroxypropyl methylcellulose HPMC and their derivatives. It should be noted that the first polysaccharide is selected from polysaccharides, that is, it can be any polysaccharide whatsoever.

Preferably, the first polysaccharide of the composition is selected from hyaluronic acid HA, starch, amylose, cellulose, chitosan, glycogen, pectin and agar.

The spinning promoter is a carrier polymer without filler selected from a group consisting of a second polysaccharide, chemically different from the first polysaccharide, possibly in the presence of poly(oxyethylene) PEO. Favorably, the carrier polymer without filler is also biocompatible. As for the first polysaccharide, the second polysaccharide is selected from polysaccharides, that is, it can be any polysaccharide whatsoever, as long as it is chemically different from the first polysaccharide. With the expression "chemically different from the first polysaccharide" we mean that the first and second polysaccharides are two molecules with different chemical structures.

Preferably, the second polysaccharide of the composition is selected from alginate and pullulan.

Preferably, the electrospinning promoter is selected from pullulan and a blend of alginate with poly(oxyethylene).

According to some embodiments, the first composition to be electrospun, and more preferably also the second composition, also comprise an active ingredient. This active ingredient can, for example, be selected from polypeptides. As previously stated, it is possible to provide that only one of either the first or the second composition comprises an active ingredient. Alternatively, it is possible to provide that the two compositions have two active ingredients, or the same active ingredient at two different concentrations.

According to some embodiments, it is possible to provide that the active ingredient is not present in the first composition to be electrospun, but that it is added to the product obtained by means of the electro spinning.

According to some embodiments, the first and possibly also the second composition comprise a stabilizer, suitable to improve the stability of the fibers obtained following the electrospinning. Preferably, the stabilizer is a cross-linkable polymer.

## Claims

1. Cosmetic patch product comprising a membrane substrate suitable to be absorbed by the skin, said membrane substrate being made up of at least one tubular shaped electrospun fiber and based on a biocompatible polymer material selected from a group consisting of hyaluronic acid HA, starch, amylose, cellulose, chitosan, glycogen, pectin, agar, collagen, hydroxypropyl methylcellulose and their derivatives, and, as an electrospinning promoter, pullulan or a blend of alginate with poly(oxyethylene).

2. Product as in claim 1, **characterized in that** the tubular fiber defines an internal containing space, and comprises a biocompatible substance in said space.

3. Product as in claim 2, **characterized in that** the biocompatible substance is electrospun.

4. Product as in any claim hereinbefore, **characterized in that** the membrane substrate is in the form of non-woven fabric.

5. Method to produce a cosmetic patch product, comprising:
providing at least a first composition to be electrospun;
providing an electrospinning device (10) comprising an electrospinning head (11) and a collector (12), wherein the electrospinning head (11) comprises a delivery member (14) comprising at least one needle (15) open at its end, the aperture of said needle (15) being annular in shape and configured to generate a tubular shaped electrospun fiber;
applying an electric field between said electrospinning head (11) and said collector (12); and
feeding said first composition to be electrospun through said electrospinning head (11), so that the electric field applied induces the formation of said tubular shaped electrospun fiber in order to form a membrane substrate suitable to be absorbed by the skin;
**characterized in that** said at least a first composition to be electrospun comprises a first compound to be electrospun and a spinning promoter, wherein said first compound to be electrospun is selected from a group consisting of hyaluronic acid HA, starch, amylose, cellulose, chitosan, glycogen, pectin, agar, collagen, hydroxypropyl methylcellulose and their derivatives, and said spinning promoter is selected from pullulan and a blend of alginate with poly(oxyethylene).

6. Method as in claim 5, **characterized in that** the delivery member (14) of the electrospinning head (11) comprises a first needle (15) and a second needle (16) with a diameter (D2) smaller than the diameter (D1) of said first needle (15), and disposed coaxial thereto, wherein the first composition to be electrospun is fed through said first needle (15).

7. Method as in claim 6, **characterized in that** it comprises:
providing a second composition;
feeding said second composition through the second needle (16) of the delivery member (14), at the same time as feeding the first composition through the first needle (15).

8. Electrospinning device, comprising an electrospinning head (11) provided with a delivery member (14), a collector (12), a voltage generator (13) connected both to said electrospinning head (11) and to said collector (12), and at least one feed element for feeding a first composition to be electrospun through said delivery member (14), wherein said delivery member (14) comprises at least a first needle (15) open at its end, the aperture of said first needle (15) being of an annular shape and configured to generate a tubular shaped electrospun fiber and a second needle (16), open at its end, the second needle (16) having a diameter (D2) smaller than the diameter (D1) of the first needle (15) and being disposed coaxial thereto, said electrospinning device (10) comprising a second feed element for feeding a second composition through said second needle (16); said electrospinning device (10) being **characterized in that** the end of the second needle (16) is disposed in a same plane as the end of the first needle (15) or in a plane downstream of the end of the first needle (15) with respect to the sense of feed of the compositions to be electrospun.

## Patentansprüche

1. Kosmetisches Pflasterprodukt, umfassend ein Membransubstrat, das zur Absorption durch die Haut geeignet ist, wobei das Membransubstrat aus mindestens einer röhrenförmigen elektrogesponnenen Faser zusammengesetzt ist und auf einem biokompatiblen Polymermaterial, ausgewählt aus der Gruppe bestehend aus Hyaluronsäure HA, Stärke, Amylose, Cellulose, Chitosan, Glykogen, Pektin, Agar, Kollagen, Hydroxypropylmethylcellulose und deren Derivaten, basiert, und als Elektrospinning-Promotor Pullulan oder eine Mischung aus Alginat mit Poly(oxyethylen).

2. Produkt nach Anspruch 1, **dadurch gekennzeichnet, dass** die röhrenförmige Faser einen inneren Hohlraum definiert und in diesem Hohlraum eine biokompatible Substanz umfasst.

3. Produkt nach Anspruch 2, **dadurch gekennzeichnet, dass** die biokompatible Substanz elektrogesponnen ist.

4. Produkt nach einem der vorstehenden Ansprüche, **dadurch gekennzeichnet, dass** das Membransubstrat die Form eines Vliesstoffs aufweist.

5. Verfahren zur Herstellung eines kosmetischen Pflasterprodukts, umfassend:
Bereitstellen mindestens einer ersten Zusammensetzung, die elektrogesponnen werden soll;
Bereitstellen einer Elektrospinnvorrichtung (10), die einen Elektrospinnkopf (11) und einen Kollektor (12) umfasst, wobei der Elektrospinnkopf (11) ein Zuführteil (14) umfasst, das mindestens eine an ihrem Ende offene Nadel (15) umfasst, wobei die Öffnung der Nadel (15) ringförmig ist und so konfiguriert ist, dass sie eine röhrenförmige elektrogesponnene Faser erzeugt;
Anlegen eines elektrischen Feldes zwischen dem Elektrospinnkopf (11) und dem Kollektor (12); und
Zuführen der ersten Zusammensetzung, die elektrogesponnen werden soll, durch den Elektrospinnkopf (11), so dass das angelegte elektrische Feld die Bildung der röhrenförmigen elektrogesponnenen Faser induziert, um ein Membransubstrat zu bilden, das zur Absorption durch die Haut geeignet ist;
**dadurch gekennzeichnet, dass** die mindestens eine erste Zusammensetzung, die elektrogesponnen werden soll, eine erste Verbindung, die elektrogesponnen werden soll, und einen Spinning-Promotor umfasst,
wobei die erste Verbindung, die elektrogesponnen werden soll, ausgewählt ist aus der Gruppe bestehend aus Hyaluronsäure HA, Stärke, Amylose, Cellulose, Chitosan, Glykogen, Pektin, Agar, Kollagen, Hydroxypropylmethylcellulose und deren Derivaten, und der Spinning-Promotor ausgewählt ist aus Pullulan und einer Mischung aus Alginat mit Poly(oxyethylen).

6. Verfahren nach Anspruch 5, **dadurch gekennzeichnet, dass** das Zuführteil (14) des Elektrospinnkopfes (11) eine erste Nadel (15) und eine zweite Nadel (16) mit einem Durchmesser (D2) umfasst, der kleiner ist als der Durchmesser (D1) der ersten Nadel (15) und koaxial zu dieser angeordnet ist, wobei die erste Zusammensetzung, die elektrogesponnen werden soll, durch die erste Nadel (15) zugeführt wird.

7. Verfahren nach Anspruch 6, **dadurch gekennzeichnet, dass** es umfasst:
Bereitstellen einer zweiten Zusammensetzung,
Zuführen der zweiten Zusammensetzung durch die zweite Nadel (16) des Zuführteils (14) zur gleichen Zeit wie das Zuführen der ersten Zusammensetzung durch die erste Nadel (15).

8. Elektrospinnvorrichtung, umfassend einen Elektrospinnkopf (11), der mit einem Zuführteil (14) ausgestattet ist, einen Kollektor (12), einen Spannungsgenerator (13), der sowohl mit dem Elektrospinnkopf (11) als auch mit dem Kollektor (12) verbunden ist, und mindestens ein Zuführungselement zum Zuführen einer ersten Zusammensetzung, die durch das Zuführteil (14) elektrogesponnen werden soll,
wobei das Zuführteil (14) mindestens eine erste Nadel (15), die an ihrem Ende offen ist, wobei die Öffnung der ersten Nadel (15) ringförmig ist und so konfiguriert ist, dass sie eine röhrenförmige elektrogesponnene Faser erzeugt, und eine zweite Nadel (16), die an ihrem Ende offen ist, wobei die zweite Nadel (16) einen Durchmesser (D2) hat, der kleiner ist als der Durchmesser (D1) der ersten Nadel (15), und koaxial zu dieser angeordnet ist, umfasst,
wobei die Elektrospinnvorrichtung (10) ein zweites Zuführungselement zum Zuführen einer zweiten Zusammensetzung durch die zweite Nadel (16) umfasst;
wobei die Elektrospinnvorrichtung (10) **dadurch gekennzeichnet ist, dass** das Ende der zweiten Nadel (16) in derselben Ebene wie das Ende der ersten Nadel (15) oder in einer Ebene stromabwärts vom Ende der ersten Nadel (15) in Bezug auf die Zuführrichtung der Zusammensetzungen, die elektrogesponnen werden sollen, angeordnet ist.

## Revendications

1. Produit de patch cosmétique comprenant un substrat membranaire pouvant être absorbé par la peau, ledit substrat membranaire étant constitué d'au moins une fibre électrofilée de forme tubulaire et à base d'un matériau polymère biocompatible choisi dans le groupe constitué par l'acide hyaluronique HA, l'amidon, l'amylose, la cellulose, le chitosane, le glycogène, la pectine, l'agar, le collagène, l'hydroxypropylméthylcellulose et leurs dérivés, et, en tant que promoteur d'électrofilage, le pullulane ou un mélange d'alginate avec du poly(oxyéthylène).

2. Produit selon la revendication 1, **caractérisé en ce que** la fibre tubulaire définit un espace de confinement interne et comprend une substance biocompatible dans ledit espace.

3. Produit selon la revendication 2, **caractérisé en ce que** la substance biocompatible est électrofilée.

4. Produit selon l'une quelconque des revendications précédentes, **caractérisé en ce que** le substrat membranaire est sous la forme d'un tissu non tissé.

5. Procédé de production d'un produit de patch cosmétique, comprenant :
la fourniture d'au moins une première composition à électrofiler ;
la fourniture d'un dispositif d'électrofilage (10) comprenant une tête d'électrofilage (11) et un collecteur (12), dans lequel la tête d'électrofilage (11) comprend un élément de distribution (14) comprenant au moins une aiguille (15) ouverte à son extrémité, l'ouverture de ladite aiguille (15) étant de forme annulaire et configurée pour générer une fibre électrofilée de forme tubulaire ;
l'application d'un champ électrique entre ladite tête d'électrofilage (11) et ledit collecteur (12) ; et
l'alimentation de ladite première composition à électrofiler à travers ladite tête d'électrofilage (11), de sorte que le champ électrique appliqué induit la formation de ladite fibre électrofilée de forme tubulaire afin de former un substrat membranaire approprié pour être absorbé par la peau ;
**caractérisé en ce que** ladite au moins une première composition à électrofiler comprend un premier composé à électrofiler et un promoteur de filage, dans lequel ledit premier composé à électrofiler est choisi dans le groupe constitué par l'acide hyaluronique HA, l'amidon, l'amylose, la cellulose, le chitosane, le glycogène, la pectine, l'agar, le collagène, l'hydroxypropylméthylcellulose et leurs dérivés, et ledit promoteur de filage est choisi parmi le pullulane et un mélange d'alginate et de poly(oxyéthylène).

6. Procédé selon la revendication 5, **caractérisé en ce que** l'élément de distribution (14) de la tête d'électrofilage (11) comprend une première aiguille (15) et une seconde aiguille (16) avec un diamètre (D2) inférieur au diamètre (D1) de ladite première aiguille (15), et disposée coaxialement à celle-ci, dans lequel la première composition à électrofiler est alimentée à travers ladite première aiguille (15).

7. Procédé selon la revendication 6, **caractérisé en ce qu'**il comprend :
la fourniture d'une seconde composition ;
l'alimentation de ladite seconde composition à travers la seconde aiguille (16) de l'élément de distribution (14), en même temps que l'alimentation de la première composition à travers la première aiguille (15).

8. Dispositif d'électrofilage, comprenant une tête d'électrofilage (11) pourvue d'un élément de distribution (14), un collecteur (12), un générateur de tension (13) relié à la fois à ladite tête d'électrofilage (11) et audit collecteur (12), et au moins un élément d'alimentation pour alimenter une première composition à électrofiler à travers ledit élément de distribution (14), dans lequel ledit élément de distribution (14) comprend au moins une première aiguille (15) ouverte à son extrémité, l'ouverture de ladite première aiguille (15) étant d'une forme annulaire et configurée pour générer une fibre électrofilée de forme tubulaire et une seconde aiguille (16), ouverte à son extrémité, la seconde aiguille (16) ayant un diamètre (D2) inférieur au diamètre (D1) de la première aiguille (15) et étant disposée coaxialement à celle-ci, ledit dispositif d'électrofilage (10) comprenant un second élément d'alimentation pour alimenter une seconde composition à travers ladite seconde aiguille (16) ; ledit dispositif d'électrofilage (10) étant **caractérisé en ce que** l'extrémité de la seconde aiguille (16) est disposée dans un même plan que l'extrémité de la première aiguille (15) ou dans un plan en aval de l'extrémité de la première aiguille (15) par rapport au sens d'alimentation des compositions à électrofiler.
